# Europäisches Patentamt
# European Patent Office
# Office européen des brevets

(11) Veröffentlichungsnummer: **0 200 136**
**B1**

(12) **EUROPÄISCHE PATENTSCHRIFT**

(45) Veröffentlichungstag der Patentschrift:
**18.01.89**

(51) Int. Cl.⁴: **A61B 6/00, A61B 6/10**

(21) Anmeldenummer: **86105491.4**

(22) Anmeldetag: **21.04.86**

(54) **Deckenstativ für einen Röntgenstrahler.**

(30) Priorität: **02.05.85 DE 8512989 U**

(43) Veröffentlichungstag der Anmeldung:
**10.12.86 Patentblatt 86/45**

(45) Bekanntmachung des Hinweises auf die Patenterteilung:
**18.01.89 Patentblatt 89/3**

(84) Benannte Vertragsstaaten:
**DE FR GB**

(56) Entgegenhaltungen:
**CH-A- 303 820**
**FR-A- 2 304 121**
**GB-A- 670 239**
**US-A- 2 909 665**
**US-A- 3 121 793**
**US-A- 3 891 856**

(73) Patentinhaber: **Siemens Aktiengesellschaft Berlin und München, Wittelsbacherplatz 2, D-8000 München 2(DE)**

(72) Erfinder: **Heinz, Lothar, Weingasse 63, D-8524 Neunkirchen a. Brand(DE)**
Erfinder: **Schmitt, Thomas, Hans-Sebald-Strasse 2, D-8550 Forchheim(DE)**

ACTORUM AG

## Beschreibung

Die Erfindung betrifft ein Deckenstativ für einen Röntgenstrahler mit einem den Röntgenstrahler tragenden Wagen, der in Schienen verschiebbar geführt ist, bei dem zur Arretieren des Wagens auf diesem ein senkrecht zur Schienenrichtung motorisch verstellbarer Reibklotz angebracht ist.

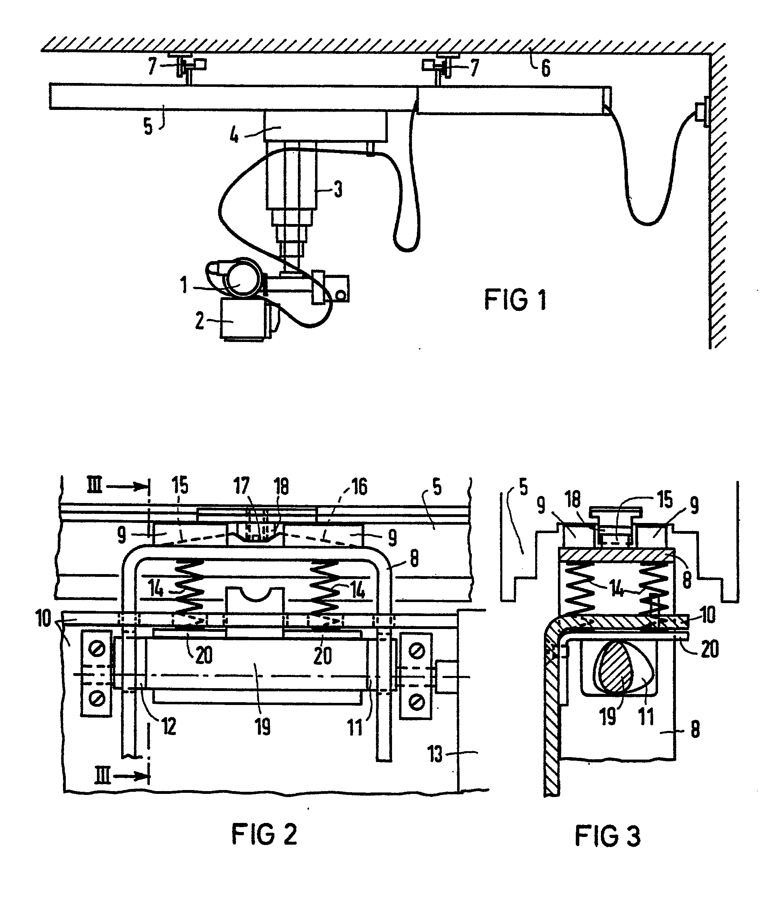

In der Figur 1 ist ein Deckenstativ dieser Art dargestellt. Ein Röntgenstrahler 1 mit einer Primärstrahlenblende 2 ist mit Hilfe eines Teleskopträgers 3 an einem Wagen 4 befestigt, der in Schienen 5 längsverschiebbar gelagert ist. Die Schienen 5 sind an senkrecht dazu verlaufenden, an der Decke 6 des Untersuchungsraumes befestigten Schienen 7 verschiebbar gelagert, so daß eine zweidimensionale Verstellung des Wagens 4 möglich ist. Aufgrund des Teleskopträgers 3 ist demgemäß der Röntgenstrahler 1 dreidimensional verstellbar. Mit Hilfe des Röntgenstrahlers 1 kann beispielsweise ein nicht dargestellter, auf einem Tisch liegender Patient in vertikaler Richtung durchstrahlt werden.

Hat der Röntgenstrahler 1 die gewünschte Stellung im Raum erreicht, so müssen der Wagen 4 in bezug auf die Schienen 5 und die Schienen 5 in bezug auf die Schienen 7 arretiert werden. Hierzu ist es bekannt, Elektromagnete vorzusehen, die mit in den Schienen 5, 7 angeordneten Leisten aus magnetisierbarem Material zusammenwirken.

Die Arretierung des Wagens 4 in bezug auf die Schienen 5 und der Schienen 5 in bezug auf die Schienen 7 kann auch durch Reibklötze erfolgen, die senkrecht zur jeweiligen Schienenrichtung motorisch verstellbar sind. Bei Anwendung solcher Reibklötze erübrigt es sich, in den den Wagen führenden Schienen magnetisierbares Material vorzusehen oder diese Schienen aus magnetisierbarem Material anzufertigen. Sie können demgemäß aus Aluminiumprofil bestehen.

Die Arretierung des Wagens erfolgt in der Regel nicht an beliebigen Stellen, sondern in Aufnahmestellungen, die sich häufig wiederholen.

Der Erfindung liegt die Aufgabe zugrunde, ein Deckenstativ der eingangs genannten Art dahingehend zu verbessern, daß bei Verwendung mindestens eines Reibklotzes zum Arretieren des Wagens, der senkrecht zur entsprechenden Schienenrichtung motorisch verstellbar ist, ein leichtes Auffinden vorbestimmter Aufnahmestellungen ermöglicht ist.

Diese Aufgabe ist erfindungsgemäß dadurch gelöst, daß der Reibklotz an einem senkrecht zur entsprechenden Schiene federnd gelagerten Halter befestigt ist, der eine der zugeordneten Schiene zugewandte Raste aufweist, die mit entsprechenden Rasten an der Schiene zusammenwirkt. Bei dem erfindungsgemäßen Deckenstativ ist es möglich, den Reibklotz motorisch so weit der Schiene anzunähern, daß der Halter beim Erreichen einer Aufnahmestellung an der Schiene einrastet und die Aufnahmestellung damit exakt definiert.

Die Erfindung ist nachfolgend anhand eines in zwei Ansichten in den Figuren 2 und 3 dargestellten Ausführungsbeispieles näher erläutert.

In den Figuren 2 und 3 ist eine der Schienen 5 dargestellt, mit der ein an einem Halter 8 befestigter Reibklotz 9 zusammenwirkt. Der Halter 8 ist dabei in einer Führung 10 geführt und durch zwei Nocken 11, 12 senkrecht zur Schiene 5 verstellbar. Die Nocken 11, 12 werden durch einen Elektromotor 13 angetrieben. Die Anpressung des Reibklotzes 9 an die Schiene 5 erfolgt durch Druckfedern 14. Der Halter 8 weist an seiner der Schiene 5 zugewandten Seite eine zwischen zwei schrägen Flächen 15, 16 liegende Aussparung 17 auf, die mit einem an der Schiene 5 befestigten Ansatz 18 zusammenwirkt.

Die Nocken 11, 12 haben gemäß Figur 3 drei Arbeitsstellungen, in die sie durch den Motor 13 verdrehbar sind. In der ersten Arbeitsstellung wird der Halter 8 mit dem Reibklotz 9 so weit von der Schiene 5 entfernt, daß der Wagen 4 völlig frei in bezug auf die Schiene 5 verstellbar ist. In der zweiten Arbeitsstellung wird der Halter 8 mit dem Reibklotz 9 so nahe an die Schiene 5 heranbewegt, daß der Wagen 4 zwar in bezug auf die Schiene 5 noch von Hand verstellbar ist, daß aber beim Überfahren des Ansatzes 18 dieser in die Aussparung 17 einrastet und eine entsprechende Aufnahmestellung definiert. In dieser Aufnahmestellung kann dann in der dritten Arbeitsstellung der Nocken 11, 12 eine Arretierung des Wagens 4 erfolgen, indem der Halter 8 so weit freigegeben wird, daß die Druckfedern 14 den Reibklotz 9 fest gegen die Schiene 5 pressen.

Zur Erhöhung der Federvorspannung beim Anpressen des Reibklotzes 9 an die Schiene 5 ist die Welle 19 zwischen den Nocken 11, 12 ebenfalls nockenförmig ausgebildet (Fig. 3) und wird durch den Motor 13 so bewegt, daß die Lagerplatte 20 für die Druckfedern 14 etwas nach oben verstellt wird und so die Spannung der Druckfedern 14 erhöht.

In der Praxis werden verschiedene Aufnahmestellungen für den Wagen 4 vorgesehen. Jeder dieser Aufnahmestellungen ist ein Ansatz an der Schiene 5 entsprechend dem Ansatz 18 zugeordnet. Eine gleichartige Rast- und Verriegelungsvorrichtung ist auch zwischen allen Schienen 5, 7 vorgesehen, so daß eine zweidimensionale Rastung und Verriegelung des Wagens 4 möglich ist.

## Patentansprüche

1. Deckenstativ für einen Röntgenstrahler (1) mit einem den Röntgenstrahler (1) tragenden Wagen (4), der in Schienen (5, 7) verschiebbar geführt ist, bei dem zum Arretieren des Wagens (4) auf diesem ein senkrecht zur Schienenrichtung motorisch verstellbarer Reibklotz (9) angebracht ist, dadurch gekennzeichnet, daß der Reibklotz (9) an einem senkrecht zur entsprechenden Schiene (5) federnd gelagerten Halter (8) befestigt ist, der eine der zugeordneten Schiene zugewandte Raste (17) aufweist, die mit entsprechenden Rasten (18) an der Schiene (5) des Wagens (4) zusammenwirkt.

2. Deckenstativ nach Anspruch 1, dadurch gekennzeichnet, daß der Halter (8) durch einen Motor (13) in drei Stellungen verstellbar ist, wobei in der ersten Stellung ein freies Bewegen des Wagens (4) möglich ist, in der zweiten Stellung beim Überfahren der Rasten (18) an der Schiene (5) eine federnde Rastung erfolgt und in der dritten Stellung der Reibklotz (9) zur Arretierung des Wagens (4) gegen die Schiene (5) gepreßt wird.

3. Deckenstativ nach Anspruch 2, dadurch gekennzeichnet, daß der Motor (13) auf die dem Halter (8) zugeordneten Federn (13, 14) zur Veränderung der Federspannung einwirkt.

## Claims

1. Ceiling mount for an X-ray tube assembly (1) having a carriage (4) which carries the X-ray source (1) and which is guided displaceably in rails (5, 7), in which for the purpose of arresting the carriage (4) there is provided on the latter a friction block (9) which can be adjusted by motor perpendicular to the direction of the rails, characterised in that the friction block (9) is secured to a holder (8) which is mounted in a resilient manner perpendicular to the corresponding rail (5) and which has a stop (17) which faces the associated rail and cooperates with corresponding stops (18) on the rail (5) of the carriage (4).

2. Ceiling mount according to claim 1, characterised in that the holder (8) can be adjusted by means of a motor (13) into three positions, wherein in the first position free movement of the carriage (4) is possible, in the second position when crossing the stops (18) on the rail (5) a resilient catching action results an in the third position the friction block (9) is pressed against the rail (5) in order to arrest the carriage (4).

3. Ceiling mount according to claim 2, characterised in that the motor (13) acts on the springs (13, 14) associated with the holder (8) in order to vary the spring tension.

## Revendications

1. Statif plafonnier pour un générateur radiologique (1) comportant un chariot (4) portant le générateur radiologique et guidé de manière à être déplaçable sur des rails (5, 7), et dans lequel un patin de friction (9) déplaçable à l'aide d'un moteur perpendiculairement à la direction du rail est prévu sur le chariot (4) pour le blocage de ce dernier, caractérisé par le fait que le patin de friction (9) est fixé à un support (8) maintenu élastiquement et perpendiculairement au rail correspondant (5), et comportant un organe d'encliquetage (17), qui est tourné vers le rail associé et coopère avec des organes d'encliquetage (18) présents sur le rail (5) de circulation du chariot (4).

2. Statif plafonnier suivant la revendication 1, caractérisé par le fait que le support (8) peut être amené au moyen d'un moteur (13) dans trois positions, auquel cas dans la première position un déplacement libre du chariot (4) est possible, dans la seconde position un encliquetage élastique se produit lors du franchissement des organes d'encliquetage (18) situés sur le rail (5) et, dans la troisième position, le patin de friction (9) est repoussé contre le rail (5) pour arrêter le chariot (4).

3. Statif plafonnier suivant la revendication 2, caractérisé par le fait que le moteur (13) agit sur les ressorts (13, 14) associés au support (8), pour modifier leur tension.

FIG 1

FIG 2

FIG 3